(19) **Europäisches Patentamt / European Patent Office / Office européen des brevets**

(11) **EP 4 702 983 A1**

(12) **EUROPEAN PATENT APPLICATION**

(43) Date of publication:
04.03.2026 Bulletin 2026/10

(21) Application number: 24382942.1

(22) Date of filing: 02.09.2024

(51) International Patent Classification (IPC):
*A61K 36/324* (2006.01)   *A61K 8/00* (2006.01)
*A61K 9/00* (2006.01)   *A61K 36/185* (2006.01)
*A61K 36/889* (2006.01)   *A61P 17/14* (2006.01)

(52) Cooperative Patent Classification (CPC):
(C-Sets available)
**A61K 36/3486; A61K 8/9789; A61K 9/00;
A61K 36/324; A61K 36/889; A61P 17/14;
A61Q 7/00** (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Lacer, S.A.U.**
**08290 Cerdanyola del Vallès, Barcelona (ES)**

(72) Inventors:
• **KILPATRICK, Lynn Eileen Campbell**
**E-08290 Cerdanyola del Vallès, Barcelona (ES)**

• **LLADÓS SEVILLA, Marta**
**E-08290 Cerdanyola del Vallès, Barcelona (ES)**
• **DE PABLO SEDANO, Marta**
**E-08290 Cerdanyola del Vallès, Barcelona (ES)**
• **RODRIGUEZ AMIGO, Beatriz**
**E-08290 Cerdanyola del Vallès, Barcelona (ES)**
• **MIRA OTAL, Francisco Javier**
**E-08290 Cerdanyola del Vallès, Barcelona (ES)**

(74) Representative: **ABG Intellectual Property Law,
S.L.**
**Avenida de Burgos, 16D
Edificio Euromor
28036 Madrid (ES)**

(54) **COMPOSITIONS FOR HAIR LOSS**

(57)    The present invention relates to synergistic compositions comprising *Humulus lupulus, Serenoa serrulata* and *Boswellia serrata* extracts, wherein the weight of *Humulus lupulus* female inflorescence extract is less than 5 times the weight of *Boswellia serrata* resin extract, and the weight of *Serenoa serrulata* fruit extract is at least 2.5 times the weight of *Boswellia serrata* resin extract, to said compositions for use in the treatment and/or prevention of alopecia, as well as to the cosmetic non-therapeutic use of said compositions to reduce hair shedding and promote hair growth.

EP 4 702 983 A1

(52)  Cooperative Patent Classification (CPC): (Cont.)

C-Sets
**A61K 36/324, A61K 2300/00;**
**A61K 36/3486, A61K 2300/00;**
**A61K 36/889, A61K 2300/00**

**Description**

**FIELD OF THE INVENTION**

The present invention relates to compositions comprising *Humulus lupulus, Serenoa serrulata* and *Boswellia serrata* extracts, to said compositions for use in the treatment and/or prevention of alopecia, as well as to the cosmetic non-therapeutic use of said compositions to reduce hair shedding and promote hair growth.

**BACKGROUND OF THE INVENTION**

The condition of the hair plays an important role in our physical appearance and self-perception. The quantity, quality, and styling of our hair define our gender, age, health, and social status. There are no significant differences in the number of hair follicles between men and women, or between the different races. Differences in the appearance of hair are due to the type of hair produced by the follicle and the type of hair care practiced by the individual.

People have between 100,000 and 150,000 hairs on their head. Losing or shedding between 50 and 100 hair strands every day is entirely natural. When the body sheds significantly more hairs every day, a person has excessive hair shedding or excessive hair loss, which is known as alopecia. Natural hair shedding and alopecia affect both males and females.

Androgenetic alopecia, which can affect both men and women, is the most common form of alopecia. It affects around 50% of men by time they reach the age of 50. In males, the hairline typically recedes and the hair becomes thin. The hair loss usually begins while a man is in his late 20s or early 30s. Alopecia can also affect women, in particular postmenopausal women. In women, hair loss generally occurs at the frontal and parietal.

Androgenic alopecia does not require treatment because it is not harmful to health. However, the hair loss can still be distressing and there are some treatment approaches available for people who becoming distressed or losing self-confidence.

Alopecia may be treated using surgical methods such as hair transplant or implants. Alternatively, a person may decide to use a wig, which may be synthetic or made of real hair.

Other treatments involve medications, such as finasteride and minoxidil, which can be used to treat male androgenic alopecia and the latter is also a treatment for female androgenic alopecia.

Dietary supplements including extracts, such as *Serenoa serrulata* extracts [Gómez-Grau et al., Rev. Argent. Dermatol,, 2015, 96(1); Mira eta I., Medicina Estética, 202, 73(4), doi 10.48158/MEDICINAESTETICA.073.03], *Humulus lupulus* extracts and *Boswellia serrata* extracts [US 2011/059192 A1] have also shown positive evidence in the treatment of alopecia or in stimulating hair growth.

Nevertheless, there is a need for further compositions or active ingredients for the treatment of alopecia and also to reduce hair loss both for cosmetic non-therapeutic purposes.

**SUMMARY OF THE INVENTION**

As shown in the examples, the inventors have surprisingly found that combining *Humulus lupulus* extract, *Serenoa serrulata* extract and *Boswellia serrata* extract at certain weight ratios results in a synergistic positive effect in oxidative stress which is correlated to hair loss.

Thus, in the first aspect, the invention relates to a composition comprising *Humulus lupulus* female inflorescence extract, *Serenoa serrulata* fruit extract and *Boswellia serrata* resin extract, wherein the weight of *Humulus lupulus* female inflorescence extract is less than 5 times the weight of *Boswellia serrata* resin extract, and the weight of *Serenoa serrulata* fruit extract is at least 2.5 times the weight of *Boswellia serrata* resin extract.

In the second aspect, the invention relates to a composition according to the first aspect for use in medicine.

In the third aspect, the invention relates to a composition according to the first aspect for use in the treatment and/or prevention of alopecia.

In the fourth aspect, the invention relates to a non-therapeutic use of a composition according to the first aspect to reduce hair shedding in a subject which does not suffer from alopecia.

In the fifth aspect, the invention relates to a non-therapeutic use of a composition according to the first aspect to promote hair growth in a subject which does not suffer from alopecia.

**DETAILED DESCRIPTION OF THE INVENTION**

The first aspect of the invention relates to a composition comprising *Humulus lupulus* female inflorescence extract, *Serenoa serrulata* fruit extract and *Boswellia serrata* resin extract, wherein the weight of *Humulus lupulus* female inflorescence extract is less than 5 times the weight of *Boswellia serrata* resin extract, and the weight of *Serenoa serrulata* fruit extract is at least 2.5 times the weight of *Boswellia serrata* resin extract.

The term "*Humulus lupulus* female inflorescence extract" is a standardized plant extract obtained from the female inflorescence of *Humulus lupulus,* a plant that is commonly known as hops, preferably from *Humulus lupulus L.* The female inflorescence of the plant is grinded, extracted with an ethanol and water mixture, in particular in a volume ratio of 1:1. The solids are then separated from the solution of the extracted constituents, e.g. by filtration or centrifugation. The solvent from the resulting solution is then removed to provide the extract. Said extract may be mixed with maltodextrin and spray dried to provide a brown powder. Typically, 55-65 wt% of the extract is mixed with 35-45 wt% of maltodextrin, preferably maize maltodextrin, wherein the wt% are expressed with respect to the total weight of extract mixture. Up to 3 wt% of silica may also be added, wherein the wt% is expressed with respect to the total weight of the extract mixture. Said extract mixture is commercially available from Givaudan/Naturex.

The term "*Serenoa serrulata* fruit extract" is a standardized plant extract obtained from the fruit of *Serenoa serrulata* a plant that is also known as *Serenoa repens* and commonly known as saw palmetto. The extract is obtained from comminuted saw palmetto fruit, in particular from dried berries, by extraction supercritical carbon dioxide, to provide an extract in the form of light to golden yellow liquid. Said extract is commercially available from SV Agrofood and/or Euromed. The extract contains from 85 to 95 wt% of C6-C20 fatty acids and from 0.2 to 0.5 wt% of sterols. Typically the fatty acid content of the extract is 8.5-24 of caproic acid, 8.5-17.5 wt% of caprylic acid, 9.0-16 wt% of capric acid, 24-30 wt% of lauric acid, 2.2-2.8 wt% of myristic acid, 2.8-3.9 wt% of palmitic acid, 14-26 wt% of stearic acid, 0.60-1.15 wt% of oleic acid, 5.0-16 wt% of linoleic acid, and 31.5-55 wt% of linolenic acid, wherein the wt% are expressed with respect to the total weight of the extract. The extract may also be mixed with dextrin, typically from 55-65 wt% of the extract is mixed with 45-35 wt% of dextrin, wherein the wt% are expressed with respect to the total weight of the extract mixture, to provide an extract mixture in the form of a light yellow powder. Said extract mixtures is commercially available from Pharmactive Biotech Products.

The term "fatty acid" refers to a saturated or unsaturated straight chain hydrocarbon having 6 or more carbon atoms in the chain, such as from 6 to 20 carbon atoms (including the carboxylic acid carbon atom), possessing a carboxyl group at one end. Examples of saturated fatty acids are caproic acid, caprylic acid, capric acid, lauric acid, myristic acid, palmitic acid, stearic acid, arachidic acid, and the like. Unsaturated fatty acids have one or more double bonds (C=C), preferably one, two, three, four, five or six double bonds. Examples of unsaturated fatty acids are myristoleic acid, palmitoleic acid, sapienic acid, oleic acid, elaidic acid, vaccenic acid, linoleic acid, linoelaidic acid, γ-linolenic acid, arachidonic acid, eicosapentaenoic acid, and the like.

The term "*Boswellia serrata* resin extract" is a standardized plant extract obtained from the resin of *Boswellia serrata,* which is commonly known as frankincense. The extract is obtained by extraction with water. The solids are then separated from the solution of the extracted constituents, e.g. by filtration. The resulting solution is then neutralized and filtrated again, if necessary. The solvent from the resulting solution is then removed to provide the extract in the form of an off white to pale yellow powder. The extract contains 60-70 wt% of boswellic acid, preferably about 65 wt%, wherein the wt% is expressed with respect to the total weight of the extract.

In the composition of the invention, the weight of *Humulus lupulus* female inflorescence extract is less than 5 times the weight of *Boswellia serrata* resin extract; preferably, the weight of *Humulus lupulus* female inflorescence extract is up to 4.5 times the weight of *Boswellia serrata* resin extract.

Preferably, in the composition of the invention, the weight of *Humulus lupulus* female inflorescence extract is at least 0.1 times the weight of *Boswellia serrata* resin extract; more preferably, weight of *Humulus lupulus* female inflorescence extract is at least the same as the weight of *Boswellia serrata* resin extract; still more the weight of *Humulus lupulus* female inflorescence extract is at least 2 times the weight of *Boswellia serrata* resin extract.

More preferably, in the composition of the invention, the ratio by weight of *Boswellia serrata* resin extract to *Humulus lupulus* female inflorescence extract is from 1:0.1 to 1:4.5; still more preferably, the ratio by weight of *Boswellia serrata* resin extract to *Humulus lupulus* female inflorescence extract is from 1:1 to 1:4.5; even more preferably, the ratio by weight of *Boswellia serrata* resin extract to *Humulus lupulus* female inflorescence extract is from 1:2 to 1:4.5.

In the composition of the invention, the weight of *Serenoa serrulata* fruit extract is at least 2.5 times the weight of *Boswellia serrata* resin extract.

Preferably, in the composition of the invention, the weight of *Serenoa serrulata* fruit extract is not more than 25 times the weight of *Boswellia serrata* resin extract; more preferably the weight of *Serenoa serrulata* fruit extract is not more than 10 times the weight of *Boswellia serrata* resin extract; still more preferably, the weight of *Serenoa serrulata* fruit extract is not more than 9 times the weight of *Boswellia serrata* resin extract.

More preferably, in the composition of the invention the ratio by weight of *Boswellia serrata* resin extract to *Serenoa serrulata* extract is from 1:2.5 to 1:24, preferably from 1:2.5 to 1:10, even more preferably from 1:2.5 to 1:9.

The composition of the invention may also comprise additional ingredients, such as maltodextrin, dextrin, silica, pumpkin seed extract, gelatin, glycerin, cystine, hydrogenated soy oil, zinc bisglycinate, zinc citrate, soy lecithin, bee wax, vitamins (such as vitamin E, vitamin B1, vitamin B2, vitamin B6, vitamin B12, vitamin D3), nicotinamide, pantothenic acid or salt thereof, sodium selenite, biotin, fructose, xanthan gum, citric acid, sodium benzoate, potassium sorbate, and water.

Preferably, the composition of the invention further comprises maltodextrin, dextrin, dextrin and/or silica.

The term "dextrin" as used herein designates a group of low-molecular-weight polymers produced by the hydrolysis of starch and glycogen. Dextrins are mixtures of polymers of D-glucose units linked by $\alpha$-(1$\rightarrow$4) or $\alpha$-(1$\rightarrow$6) glycosidic bonds.

The term "maltodextrin" as used herein refers to a polymer consisting of D-glucose units connected in chains of variable length. It is a short-chain polymer; e.g. having an average of 2 to 20 glucose units per chain. The glucose units are primarily linked with $\alpha(1\rightarrow4)$ glycosidic bonds.

The term "silica" as used herein refers to silicon dioxide.

Preferably, the compositions of the present invention are intended for oral or topical administration. Thus, the compositions of the invention are preferably in a form suitable for oral administration or topical application, such as capsules, solutions, suspension, gummies, lotions, and the like.

The term "topical" is used herein to characterize a composition as being suitable for application in the exterior of the body such as, without limitation, the hair, scalp and skin.

The term "oral" is used herein to characterize a composition as being suitable for administration through the mouth, swallowing and then processed vie the digestive system.

In a particular embodiment, the composition of the invention comprises:

- from 0.001 to 0.01 wt% of *Humulus lupulus* female inflorescence extract,
- from 0.025 to 0.05 wt% of *Serenoa serrulata* fruit extract,
- from 0.0025 to 0.01 wt% of *Boswellia serrata* resin extract,

wherein the wt% are expressed with respect to the total weight of the composition and wherein the weigh ratios of the extracts are as previously defined.

In another particular embodiment, the compositions of the invention for oral administration comprises:

- from 0.001 to 10 wt% of Humulus lupulus female inflorescence extract,
- from 0.025 to 20 wt% of Serenoa serrulata fruit extract,
- from 0.0025 to 5 wt% of *Boswellia serrata* resin extract,

wherein the wt% are expressed with respect to the total weight of the composition and wherein the weigh ratios of the extracts are as previously defined.

In another particular embodiment, the compositions of the invention for topical administration comprises:

- from 0.001 to 0.5 wt% of *Humulus lupulus* female inflorescence extract,
- from 0.025 to y 1 wt% of *Serenoa serrulata* fruit extract,
- from 0.0025 to y 0.3 wt% of *Boswellia serrata* resin extract,

wherein the wt% are expressed with respect to the total weight of the composition and wherein the weigh ratios of the extracts are as previously defined.

The compositions of the invention may be prepared by conventional means well known to the skilled person, such as blending the components to provide the target composition.

The invention also relates to a pharmaceutical composition comprising the composition of the invention and a pharmaceutically acceptable excipient.

The term "pharmaceutically acceptable excipient" refers to a vehicle, diluent, or adjuvant that is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similars. Suitable pharmaceutical vehicles are described in "Remington's Pharmaceutical Sciences" by E.W. Martin, 21st Edition, 2005.

The invention also relates to a nutraceutical product or cosmetic composition comprising the composition of the invention.

As used herein, the term "nutraceutical product" refers to a product suitable for use in human beings or animals, such as dietary supplements presented in a non-food matrix (e.g., capsules, powder, etc.). Therefore, the term "nutraceutical product" includes food supplements which are generally presented in dosage forms normally used orally, for example, capsules, drinkable products (such as solutions or suspensions), gummies, tablets, sachets, etc.

The term "cosmetic composition", as used herein, refers to a composition suitable for use in personal hygiene of human beings or animals, or in order to enhance the natural beauty or change the body appearance without affecting the structure or functions of the human or animal body which comprises the composition of the invention and a cosmetically acceptable excipient. If desired, the cosmetic composition provided by the invention can contain, in addition to the composition of the invention, one or more cosmetics or cosmetic products, i.e., substances or mixtures intended to be placed in contact with the external parts of the human or animal body (e.g., hair, scalp, epidermis, etc.), for the exclusive or main purpose of changing their appearance, or keeping them in good condition. Illustrative examples of cosmetically acceptable excipient include the products contained in the INCI (International Nomenclature of Cosmetic Ingredients) list. Cosmetic compositions include products such as lotions, balms, pads, pomades, creams, etc. oils, surfactants, humectants, botanical extracts, vitamins, antioxidants, sunscreen agents, perfumes, preservatives, and the like. The resulting cosmetic composition may be in the form of a liquid, solid, semi-solid, dispersion, suspension, solution or emulsion, and it can be either aqueous-based or anhydrous.

As shown in the examples, the compositions of the invention have a synergistic positive effect on oxidative stress which correlates to hair loss, as explained below.

Oxidative stress, the inability of the body to sufficiently counteract the sources of oxidation, is prevalent in many skin conditions, including normal skin aging. On the scalp, the hair appears to be impacted prior to emergence, and oxidative stress appears to play a role in premature hair loss [Trüeb R.M. et al., Int. J. Trichology, 2018, 10(6), 262-270]. It has been reported that oxidative stress correlates with changes in hair follicles leading to hair loss in both men and women [Ibrahim YA. et al., Benha Journal of Applied Sciences, 2023, 8(12), 105-110]. It has also been reported that the main cause of premature dermal papilla scalp cell aging is oxidative stress and the gradual accumulation of reactive oxygen species (ROS) in cells, resulting in the loss of their function. ROS mediates responses to dihydrotestosterone (DHT) that stimulates the secretion of hair growth inhibitory factors in men [Cwynar A. et al., Postepy Dermatol Alergol., 2020, 37(1), 119-120]. Also, in women, the action of ROS results in damage to cellular components, such as nucleic acids, proteins and lipids of the cell membrane. In particular, an increase in lipid peroxidation has been observed in female patients suffering from androgenic alopecia due to the presence of oxidative stress [Cwynar A. et al., Postepy Dermatol Alergol., 2020, 37(1), 119-120].

Thus, in the second aspect, the invention relates to a composition according to the first aspect for use in medicine.

In a third aspect, the invention relates to a composition according to the first aspect for use in the treatment and/or prevention of alopecia.

This aspect may also be formulated as the use of a composition according to the first aspect in the manufacture of a medicament for the treatment and/or prevention of alopecia.

Alternatively, this aspect may be formulated as a method for the treatment and/or prevention of alopecia in a subject in need thereof, comprising administering the composition according to the first aspect.

The term "treatment", as used herein, refers to any type of therapy, which is aimed at terminating, ameliorating or reducing the susceptibility to a clinical condition as described herein. Thus, "treatment," "treating," and their equivalent terms refer to obtaining a desired pharmacologic or physiologic effect, covering any treatment of a pathological condition or disorder in a mammal, including a human. The effect may be a partial or complete cure for a disorder and/or adverse effect attributable to the disorder. That is, "treatment" includes inhibiting the disorder, such as arresting its development, stopping or terminating the disorder or, at least, symptoms associated therewith, so that the host no longer suffers from the disorder or its symptoms, such as causing regression of the disorder or its symptoms, for example, by restoring or repairing a lost, missing or defective function, or stimulating an inefficient process, or relieving, alleviating, or ameliorating the disorder, or symptoms associated therewith, where ameliorating is used in a broad sense to refer to at least a reduction in the magnitude of a parameter.

The term "prevention", "preventing" or "prevent", as used herein, relates to the administration of a composition according to the invention or of a medicament comprising said composition to a subject who has not been diagnosed as possibly having the disease, but who would normally be expected to develop said disease or be at increased risk for said disease. The prevention intends to avoid the appearance of said disease. The prevention may be complete (e.g. the total absence of a disease). The prevention may also be partial, such that for example the occurrence of a disease in a subject is less than that which would have occurred without the administration of the composition of the present invention. Prevention also refers to reduced susceptibility to a clinical condition.

In a preferred embodiment, the alopecia is androgenic alopecia.

In a particular embodiment, the androgenic alopecia is female androgenic alopecia.

In another particular embodiment, the androgenic alopecia is male androgenic alopecia.

Due to the positive synergistic effects found by the inventors, as shown in the examples, the compositions of the invention may also be used to reduce hair shedding and/or promote hair growth in subjects who do not suffer from alopecia.

Thus, in the fourth aspect, the invention relates to a composition according to the first aspect to reduce hair shedding in a subject which does not suffer from alopecia.

The expression "reduce hair shedding" refers to a reduction in at least 5%, preferably at least 10%, more preferably at least 20%, more preferably at least 30%, more preferably at least 40%, still more preferably at least 50% of the amount of hair lost by the subject before the use of the composition according to the invention.

In the fifth aspect, the invention relates a non-therapeutic use of a composition according to the first aspect to promote hair growth in a subject which does not suffer from alopecia.

The expression "promote hair growth" refers to an increase of at least 5%, preferably at least 10%, more preferably at least 20%, more preferably at least 30%, more preferably at least 40%, still more preferably at least 50% of the number of new hair strands in subject before the use of the composition according to the invention.

When using the compositions of the invention for the therapeutic and non-therapeutic applications, said compositions may be administered by any suitable route, such as the oral and topical routes.

The following examples are merely illustrative of certain embodiments of the invention and cannot be considered as restricting it in any way.

**Examples**

**Example 1. In vitro activity study**

Materials and methods

Oxidative stress is correlated to hair loss [Trüeb R.M. et al., Int. J. Trichology, 2018, 10(6), 262-270; Ibrahim Y.A. et al., Benha Journal of Applied Sciences, 2023, 8(12), 105-110; and Cwynar A. et al., Postepy Dermatol Alergol., 2020, 37(1), 119-120]. Thus, the antioxidant activity has been used as a suitable *in vitro* model for determining the activity of the compositions of the invention on hair loss.

Antioxidant activity was studied by 2',7'-difluorodihydrofluorescein diacetate ($H_2$DFFDA), a method used to determine production of reactive oxygen species (ROS). $H_2$DFFDA permeates the cell membrane and accumulates mostly in the cytosol. $H_2$DFFDA is then deacetylated by cellular esterases to non-fluorescent difluorodihydrofluorescein ($H_2$DFF). This non-fluorescent product is converted by ROS into the fluorescent and detectable difluorofluorescein (DFF). Two independent assays were carried out, using six technical replicates in each of them.

15,000 immortalized human hair follicle dermal papilla cells were seeded in 96 well-plates in complete media and cultured overnight at 37 °C, 5% $CO_2$. Next, cells were incubated with 50 μM) $H_2$DFFDA for 45 mins. After incubation, cells were washed with PBS and exposed to 250 μM tBHP (*tert*-butyl hydroperoxide) (ROS-generating agent) in presence of the test individual extracts *Humulus lupulus* female inflorescence extract (Givaudan/Naturex ref. EA149331) (HL), *Serenoa serrulata* fruit extract (Euromed. Ref. 890045) (SS) and *Boswellia serrata* gum extract (Natural Extract ref. BOSW65003) (BS) and compositions comprising said extracts at several concentration ratios. To prepare these test compositions, the extracts were first dissolved in 100% DMSO and then diluted in Hanks' Balanced Salt Solution so that the DMSO concentration did not exceed 0.5% concentration in any well. Ascorbic acid 10 μg/ml was used as a positive control.

After an incubation period of 4 hours fluorescence was measured in a microplate reader (Ex=485 nm; Em= 535nm). The percentage of oxidative stress was calculated relative to the fluorescence values of the negative control (C-tBHP), a condition in which cells were not treated with any compound, by applying the following formula:

$$Oxidative\ stress\ (\%) = \left[ \frac{\left( F^{485/535}{}_{CX} \right) \times 100}{F^{485/535}{}_{C-}} \right]$$

$F^{485/535}$ cx: fluorescence at Ex/Em = 485/535 nm of the wells treated with the test compounds at a given concentration.

$F^{485/535}$ c-: fluorescence at Ex/Em = 485/535 nm of the wells not treated with any compound (C- tBHP).

Results

The results for the extract combinations that have shown a synergistic antioxidant activity are gathered in Table 1 below, including also the antioxidant activity of the individual extracts.

Table 1. Antioxidant activity values of the synergistic combinations.

| | Concentration (% w/v) | Antioxidant activity (% relative to C-tBHP) | SD | Summary individual compounds |
|---|---|---|---|---|
| C- | healthy | 35.69 | 5.61 | |
| | tBHP | 0.00 | 9.39 | |
| C+ | ascorbic acid | 64.75 | 3.76 | |

(continued)

| | Concentration (% w/v) | Antioxidant activity (% relative to C-tBHP) | SD | Summatory individual compounds |
|---|---|---|---|---|
| Combinations | HL 0.01% + SS 0.025% + BS 0.005% Ratio HL/BS: 2 Ratio SS/BS: 5 | 70.24 | 9.40 | 65.18 |
| | HL 0.01% + SS 0.025% + BS 0.0025% Ratio HL/BS: 4 Ratio SS/BS: 10 | 72.48 | 3.77 | 66.07 |
| | HL 0.005% + SS 0.05% + BS 0.01% Ratio HUBS: 0.5 Ratio SS/BS: 5 | 67.61 | 3.02 | 64.72 |
| | HL 0.005% + SS 0.05% + BS 0.005% Ratio HL/BS: 1 Ratio SS/BS: 10 | 63.19 | 4.02 | 60.76 |
| | HL 0.005% + SS 0.025% + BS 0.01% Ratio HUBS: 0.5 Ratio SS/BS: 2.5 | 67.65 | 4.42 | 60.02 |
| | HL 0.005% + SS 0.025% + BS 0.005% Ratio HL/BS: 1 Ratio SS/BS: 5 | 59.57 | 5.45 | 56.06 |
| | HL 0.001% + SS 0.025% + BS 0.01% Ratio HUBS: 0.1 Ratio SS/BS: 2.5 | 35.02 | 9.49 | 29.58 |
| | HL 0.001% + SS 0.025% + BS 0.005% Ratio HUBS: 0.2 Ratio SS/BS: 5 | 26.99 | 5.13 | 25.62 |
| | HL 0.001% + SS 0.025% + BS 0.0025% Ratio HUBS: 0.4 Ratio SS/BS: 10 | 30.09 | 10.38 | 26.51 |
| | HL 0.001% + SS 0.05% + BS 0.01% Ratio HUBS: 0.1 Ratio SS/BS: 5 | 44.75 | 7.00 | 34.28 |
| HL | 0.01 | 56.84 | 6.42 | |
| | 0.005 | 47.72 | 6.54 | |
| | 0.001 | 17.28 | 5.91 | |
| SS | 0.05 | 4.71 | 4.70 | |
| | 0.025 | 0.01 | 5.28 | |
| BS | 0.01 | 12.29 | 8.79 | |
| | 0.005 | 8.33 | 4.07 | |
| | 0.0025 | 9.22 | 3.46 | |

The results for the extract combinations that have not shown a synergistic antioxidant activity are gathered in Table 2 below, including also the antioxidant activity of the individual extracts.

8

Table 2. Antioxidant activity values of the non-synergistic combinations.

| | Concentration (% w/v) | Antioxidant activity (% relative to C- tBHP) | SD | Summatory individual compounds |
|---|---|---|---|---|
| Combinations | HL 0.1% + SS 0.05% + BS 0.01%<br>Ratio HL/BS: 10<br>Ratio SS/BS: 5 | 83.03 | 1.29 | 94.00 |
| | HL0.1% + SS 0.05%+ BS 0.005%<br>Ratio HL/BS: 20<br>Ratio SS/BS: 10 | 80.47 | 3.20 | 90.04 |
| | HL 0.1% + SS 0.01% + BS 0.01%<br>Ratio HL/BS: 10<br>Ratio SS/BS: 1 | 83.47 | 2.84 | 90.74 |
| | HL 0.1% + SS 0.01% + BS 0.005%<br>Ratio HL/BS: 20<br>Ratio SS/BS: 2 | 78.39 | 1.54 | 86.78 |
| | HL 0.05% + SS 0.05% + BS 0.01%<br>Ratio HL/BS: 5<br>Ratio SS/BS: 5 | 78.07 | 2.50 | 83.23 |
| | HL 0.05% + SS 0.05% + BS 0.005%<br>Ratio HL/BS: 10<br>Ratio SS/BS: 10 | 77.94 | 0.82 | 79.27 |
| | HL 0.05% + SS 0.025% + BS 0.01%<br>Ratio HL/BS: 5<br>Ratio SS/BS: 2.5 | 78.34 | 3.66 | 78.53 |
| | HL 0.05% + SS 0.025% + BS 0.005%<br>Ratio HL/BS: 10<br>Ratio SS/BS: 5 | 74.48 | 3.63 | 74.57 |
| | HL 0.005% + SS 0.01% + BS 0.01%<br>Ratio HUBS: 0.5<br>Ratio SS/BS: 1 | 60.36 | 4.82 | 61.46 |
| | HL 0.005% + SS 0.01% + BS 0.005%<br>Ratio HL/BS: 1<br>Ratio SS/BS: 2 | 51.73 | 13.43 | 57.50 |
| HL | 0.1 | 77.00 | 4.55 | |
| | 0.05 | 66.23 | 2.11 | |
| | 0.005 | 47.72 | 6.54 | |
| SS | 0.05 | 4.71 | 4.70 | |
| | 0.025 | 0.01 | 5.28 | |
| | 0.01 | 1.45 | 0.83 | |
| BS | 0.01 | 12.29 | 8.79 | |
| | 0.005 | 8.33 | 4.07 | |

As shown in the results gathered in the tables above, the *Humulus lupulus* female inflorescence extract, *Serenoa serrulata* fruit extract and *Boswellia serrata* gum extract when combined so that the weight of *Humulus lupulus* female inflorescence extract is less than 5 times the weight of *Boswellia serrata* resin extract and the weight of *Serenoa serrulata* fruit extract is at least 2.5 times the weight of *Boswellia serrata* gum extract have a synergistic antioxidant effect, whereas said synergy is not observed when combining the extracts at other weight ratios.

**Example 2. Formulations**

Oral and topical formulations are prepared with the ingredients listed below:
Capsules have the following ingredients: Pumpkin seed extract (400 mg/capsule) (*Cucurbita pepo L.*), Gelatin, L-Cystine (300 mg/capsule), *Serenoa serrulata* fruit extract (280 mg/capsule), Humectant (Glycerin), *Humulus lupulus* female inflorescence extract (100 mg/capsule), Hydrogenated soya oil, Zinc (10 mg/capsule) (Bisglicinato de zinc), Emulgent (Soy lecithin), Thickener (Yellow bee wax), *Boswellia serrata* resin extract (25 mg/capsule), Vitamin E (12 mg/capsule) (DL-alpha-tocopheryl acetate), Niacin (16 mg/capsule) (Nicotinamide), Pantothenic acid (6.0 mg/capsule) (Calcium D-pantothenate), Colorant (Iron oxides and hydroxides), Vitamin B12 (2.5 μg/capsule) (Cyanocobalamin), Vitamin B6 (1.4 mg/capsule) (Pyridoxine hydrochloride), Riboflavin (1.4 mg/capsule) (Vitamin B2), Thiamine (1.1 mg/capsule) (Thiamine mononitrate), Vitamin D (5.0 μg/capsule) (Cholecalciferol), Selenium (55 μg/capsule) (Sodium selenite), and Biotin (50 μg/capsule) (D-biotin).

Oral suspensions have the following ingredients: Water, Fructose, *Serenoa serrulata* fruit extract (560 mg/bottle), Pumpkin seed extract (*Cucurbita pepo L.*) (400 mg/bottle), L-Cystine (300 mg/bottle), Flavours, *Humulus lupulus* female inflorescence extract (100 mg/bottle) , Zinc (10 mg/bottle) (Zinc bisglycinate), Vitamin E (12 mg/bottle) (DL-alpha-tocopheryl acetate), Thickener (Xanthan gum), *Boswellia serrata* resin extract (25 mg/bottle), Acidulant (Citric acid), Niacin (16 mg/bottle) (Nicotinamide), Preservatives (Sodium benzoate and Potassium sorbate), Pantothenic acid (6.0 mg/bottle) (Calcium D-pantothenate), Vitamin B12 (2.5 μg/bottle) (Cyanocobalamin), Vitamin D (5.0 μg/bottle) (Cholecalciferol), Vitamin B6 (1.4 mg/bottle) (Pyridoxine hydrochloride), Riboflavin (1.4 mg/bottle) (Vitamin B2), Thiamine (1.1 mg/bottle) (Thiamine mononitrate), Selenium (55 μg/bottle) (Sodium selenite) and Biotin (50 μg/bottle) (D-biotin).

Cosmetic lotions have the following ingredients: Alcohol Denat., Water, Niacinamide, PEG-40 Hydrogenated Castor Oil, Ethoxydiglycol, Propanediol, Glycerin, Caffeine, *Serenoa Serrulata* Fruit Extract, Tocopheryl Acetate, Dipotassium Glycyrrhizate, Panthenol, Pyridoxine HCl, Dipropylene Glycol, Zinc PCA, Sodium Citrate, *Humulus Lupulus* Female Inflorescence Extract, *Boswellia Serrata* Resin Extract and Citric Acid.

**Claims**

1. A composition comprising *Humulus lupulus* female inflorescence extract, *Serenoa serrulata* fruit extract and *Boswellia serrata* gum extract, wherein the weight of *Humulus lupulus* female inflorescence extract is less than 5 times the weight of *Boswellia serrata* resin extract, and the weight of *Serenoa serrulata* fruit extract is at least 2.5 times the weight of *Boswellia serrata* gum extract.

2. A composition according to claim 1, wherein the weight of *Humulus lupulus* female inflorescence extract is at least 0.1 times the weight of *Boswellia serrata* resin extract.

3. A composition according to claim 1 or 2, wherein the ratio by weight of *Boswellia serrata* resin extract to *Humulus lupulus* female inflorescence extract is from 1:0.1 to 1:4.5.

4. A composition according to claim 3, wherein the ratio by weight of *Boswellia serrata* resin extract to *Humulus lupulus* female inflorescence extract is from 1:1 to 1:4.5, preferably from 1:2 to 1:4.5.

5. A composition according to any one of the preceding claims, wherein the weight of *Humulus lupulus* female inflorescence extract is at least 2 times the weight of *Boswellia serrata* resin extract.

6. A composition according to any one of the preceding claims, wherein the weight of *Serenoa serrulata* fruit extract is not more than 25 times the weight of *Boswellia serrata* resin extract.

7. A composition according to any one of the preceding claims, wherein the ratio by weight of *Boswellia serrata* resin extract to *Serenoa serrulata* fruit extract is from 1:2.5 to 1:10, preferably from 1:2.5 to 1:9.

8. Composition according to any one of the preceding claims, further comprising maltodextrin, dextrin and/or silica.

9. Composition according to any one of the preceding claims in a form suitable for oral administration or topical application.

10. Composition according to any one of the preceding claims in a form selected from the group consisting of capsules, solution, suspension, gummies and lotion.

**11.** Composition according to any one of the preceding claims for use in medicine.

**12.** Composition according to any one of the preceding claims for use in the treatment and/or prevention of alopecia.

**13.** Composition for use according to claim 12, wherein alopecia is androgenic alopecia.

**14.** Non-therapeutic use of a composition according to any one of claims 1 to 10 to reduce hair shedding in a subject which does not suffer from alopecia.

**15.** Non-therapeutic use of a composition according to any one of claims 1 to 10 to promote hair growth in a subject which does not suffer from alopecia.

Europäisches
Patentamt

European
Patent Office

Office européen
des brevets

## EUROPEAN SEARCH REPORT

| | Application Number |
|---|---|
| | **EP 24 38 2942** |

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/216986 A1 (POHLMANN ADRIANA RAFFIN [BR] ET AL) 6 August 2015 (2015-08-06) * the whole document * | 1-15 | INV. A61K36/324 A61K8/00 A61K9/00 |
| X | US 2011/059192 A1 (GLYNN KELLY M [US] ET AL) 10 March 2011 (2011-03-10) * the whole document * | 1-15 | A61K36/185 A61K36/889 A61P17/14 |
| X | US 2007/036742 A1 (ROUFS JAMES B [US] ET AL) 15 February 2007 (2007-02-15) * the whole document * | 1-15 | |
| X | DATABASE GNPD [Online] MINTEL; 28 December 2011 (2011-12-28), anonymous: "Root Power Restorative Style Wax", XP093242078, Database accession no. 1701503 * abstract * | 1-15 | |

| | TECHNICAL FIELDS SEARCHED (IPC) |
|---|---|
| | A61K A61Q A61P |

The present search report has been drawn up for all claims

| Place of search | Date of completion of the search | Examiner |
|---|---|---|
| Munich | 23 January 2025 | Friederich, Martin |

EPO FORM 1503 03.82 (P04C01)

## ANNEX TO THE EUROPEAN SEARCH REPORT
## ON EUROPEAN PATENT APPLICATION NO.

EP 24 38 2942

This annex lists the patent family members relating to the patent documents cited in the above-mentioned European search report.
The members are as contained in the European Patent Office EDP file on
The European Patent Office is in no way liable for these particulars which are merely given for the purpose of information.

23-01-2025

| Patent document cited in search report | Publication date | Patent family member(s) | | Publication date |
|---|---|---|---|---|
| US 2015216986 A1 | 06-08-2015 | AR | 092403 A1 | 22-04-2015 |
| | | AU | 2013308356 A1 | 16-04-2015 |
| | | BR | 102012022036 A2 | 25-11-2014 |
| | | CA | 2883998 A1 | 06-03-2014 |
| | | CL | 2015000501 A1 | 03-07-2015 |
| | | CN | 104755075 A | 01-07-2015 |
| | | CO | 7310520 A2 | 30-06-2015 |
| | | DK | 2891486 T3 | 23-07-2018 |
| | | EP | 2891486 A1 | 08-07-2015 |
| | | ES | 2683418 T3 | 26-09-2018 |
| | | JP | 6352265 B2 | 04-07-2018 |
| | | JP | 2015526484 A | 10-09-2015 |
| | | KR | 20150044029 A | 23-04-2015 |
| | | MX | 363961 B | 09-04-2019 |
| | | MY | 174692 A | 07-05-2020 |
| | | PE | 20150732 A1 | 17-05-2015 |
| | | PE | 20191714 A1 | 05-12-2019 |
| | | TR | 201810389 T4 | 27-08-2018 |
| | | UA | 113774 C2 | 10-03-2017 |
| | | US | 2015216986 A1 | 06-08-2015 |
| | | UY | 35002 A | 31-03-2014 |
| | | WO | 2014032152 A1 | 06-03-2014 |
| | | ZA | 201501255 B | 27-01-2016 |
| US 2011059192 A1 | 10-03-2011 | NONE | | |
| US 2007036742 A1 | 15-02-2007 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2011059192 A1

**Non-patent literature cited in the description**

- **GÓMEZ-GRAU et al.** *Rev. Argent. Dermatol,*, 2015, vol. 96 (1)
- **MIRA**. *Medicina Estética*, vol. 73 (4)
- **E.W. MARTIN**. Remington's Pharmaceutical Sciences. 2005
- **TRÜEB R.M. et al.** *Int. J. Trichology,* 2018, vol. 10 (6), 262-270
- **IBRAHIM YA. et al.** *Benha Journal of Applied Sciences*, 2023, vol. 8 (12), 105-110
- **CWYNAR A. et al.** *Postepy Dermatol Alergol.*, 2020, vol. 37 (1), 119-120
- **IBRAHIM Y.A. et al.** *Benha Journal of Applied Sciences*, 2023, vol. 8 (12), 105-110